# EUROPÄISCHE PATENTSCHRIFT

(11) **EP 2 774 571 B1**
(45) Veröffentlichungstag und Bekanntmachung des Hinweises auf die Patenterteilung: **11.07.2018**
(21) Anmeldenummer: 13158142.3
(22) Anmeldetag: 07.03.2013
(51) Int. Cl.: A61C 1/05, H02K 1/27, F03B 13/04, H02K 21/12

(54) **Dynamoelektrischer Wandler und medizinische, insbesondere dentale, Vorrichtung mit einem dynamoelektrischen Wandler**
Dynamoelectric converter and medical, in particular dental device with a dynamoelectric converter
Convertisseur dynamoélectrique et dispositif médical, en particulier dentaire, doté d'un convertisseur dynamoélectrique

(43) Veröffentlichungstag der Anmeldung: 10.09.2014
(73) Patentinhaber: W & H Dentalwerk Bürmoos GmbH, 5111 Bürmoos (AT)
(72) Erfinder: Hofbauer, Manfred, 5111 Bürmoos (AT); Schmiedlechner, Karl, 5121 Ostermiething (AT); Schuh, Walter, 5111 Bürmoos (AT); Schmitzberger, Thomas, 5122 Hochburg-Ach (AT)
(74) Vertreter: Benda, Ralf

(56) Entgegenhaltungen:
- EP-A1- 2 704 295
- WO-A1-2011/043143
- FR-A1- 2 523 891
- US-A1- 2012 115 101

## Beschreibung

Die vorliegende Erfindung betrifft einen fluidbetriebenen, dynamoelektrischen Wandler zur Erzeugung elektrischer Energie und eine medizinische, insbesondere dentale, Vorrichtung mit einem fluidbetriebenen, dynamoelektrischen Wandler.

Die Patentanmeldung US 2008/261172 A1 offenbart einen fluidbetriebenen, dynamoelektrischen Wandler zur Erzeugung elektrischer Energie und eine medizinische, insbesondere dentale, Vorrichtung in Form eines Handstücks oder eines Kupplungselements mit einem fluidbetriebenen, dynamoelektrischen Wandler. Das Vorsehen eines fluidbetriebenen, dynamoelektrischen Wandlers in einem Handstück oder in einem Kupplungselement ermöglicht es, einen elektrischen Verbraucher in dem Handstück oder in dem Kupplungselement, insbesondere eine Beleuchtungsvorrichtung, mit elektrischer Energie des dynamoelektrischen Wandler zu versorgen, ohne dass das Handstück oder das Kupplungselement mit einer elektrischen Energiequelle verbunden ist. Derartige, von der Anmelderin seit mehreren Jahren erzeugte und vertriebene Handstücke oder Kupplungselemente mit fluidbetriebenen, dynamoelektrischen Wandler werden von den Anwendern außerordentlich geschätzt. Die Dokumente FR253891 und US2012/0115101 offenbaren auch fluidbetriebene dynamoelektrische Wandler.

Der vorliegenden Erfindung liegt die Aufgabe zugrunde einen fluidbetriebenen, dynamoelektrischen Wandler für eine medizinische, insbesondere dentale, Vorrichtung weiter zu entwickeln. Insbesondere sollen der Aufbau des fluidbetriebenen, dynamoelektrischen Wandlers vereinfacht, seine Abmessungen verringert und die Integration oder der Einbau in die medizinische, insbesondere dentale, Vorrichtung erleichtert werden, ohne dabei Einbußen der elektrischen Leistung des dynamoelektrischen Wandlers zu verursachen.

Diese Aufgabe wird durch eine medizinische, insbesondere dentale, Vorrichtung mit einem dynamoelektrischen Wandler (im Folgenden auch als Generatoren bezeichnet) nach Anspruch 1 gelöst.

Es ist eine medizinische, insbesondere dentale, Vorrichtung mit einem lösbar mit einer Fluidquelle verbindbaren Anschlussende und mit einem fluidbetriebenen dynamoelektrischen Wandler vorgesehen, wobei der fluidbetriebene dynamoelektrische Wandler umfasst: Einen Rotor mit einem durch ein Fluid in Drehung versetzbaren Laufrad und zumindest einem Magnetelement, das mit dem Laufrad verbunden ist, so dass das Magnetelement durch das in Drehung versetzbare Laufrad ebenfalls in Drehung versetzbar ist, einen Stator mit einer elektrischen Wicklung, die mit dem in Drehung versetzbaren Magnetelement derart zusammenwirkt, dass in der elektrischen Wicklung elektrische Energie erzeugbar ist, eine Hülse, die zumindest Teile des Rotors und / oder des Stators umgibt, und eine einteilig mit der Hülse des dynamoelektrischen Wandlers ausgebildete Fluidleitung, welche zumindest mit ihrem von dem dynamoelektrischen Wandler abgewandten Ende über das Anschlussende der medizinischen, insbesondere dentalen, Vorrichtung ragt.

Gemäß einem Ausführungsbeispiel ist ein fluidbetriebener, dynamoelektrischer Wandler vorgesehen, der umfasst: Einen Rotor mit einem durch ein Fluid in Drehung versetzbaren Laufrad mit mehreren Schaufeln, zumindest einem Magnetelement, das mit dem Laufrad verbunden ist, so dass das Magnetelement durch das in Drehung versetzbare Laufrad ebenfalls in Drehung versetzbar ist, und mit einer Rotorhülse, in welcher das zumindest eine Magnetelement des Rotors angeordnet ist, und einen Stator mit einer elektrischen Wicklung, die mit dem in Drehung versetzbaren Magnetelement derart zusammenwirkt, dass in der elektrischen Wicklung elektrische Energie erzeugbar ist, wobei das Laufrad, insbesondere die Schaufeln des Laufrads, einteilig mit der Rotorhülse ausgebildet ist / sind und insbesondere an einer Außenseite der Rotorhülse und / oder an einem Ende der Rotorhülse angeordnet ist / sind.

Gemäß einem weiteren Ausführungsbeispiel ist ein fluidbetriebener, dynamoelektrischer Wandler vorgesehen, der umfasst: Einen Rotor mit einem durch ein Fluid in Drehung versetzbaren Laufrad und zumindest einem Magnetelement, das mit dem Laufrad verbunden ist, so dass das Magnetelement durch das in Drehung versetzbare Laufrad ebenfalls in Drehung versetzbar ist, und einen Stator mit einer elektrischen Wicklung, die mit dem in Drehung versetzbaren Magnetelement derart zusammenwirkt, dass in der elektrischen Wicklung elektrische Energie erzeugbar ist, und mit einem magnetischen Rückschlusselement zum Konzentrieren und Lenken der magnetischen Feldlinien des zumindest einen Magnetelements durch die elektrische Wicklung. Das magnetische Rückschlusselement weist ein einteiliges, hülsenförmiges, magnetisches Rückschlusselement, das den Rotor und die elektrische Wicklung umgibt und das ein magnetisch leitendes Material umfasst, auf oder ist als selbiges ausgebildet.

Die einteilige Ausbildung von Bauteilen bewirkt eine einfachere Integration des fluidbetriebenen, dynamoelektrischen Wandlers in die medizinische, insbesondere dentale Vorrichtung und / oder einen geringeren Platzverbrauch im Inneren der medizinischen, insbesondere dentalen Vorrichtung. Aufgrund des geringeren Platzverbrauchs ist es somit des Weiteren in vorteilhafter Weise möglich, andere Bauteile der medizinischen, insbesondere dentalen Vorrichtung, zum Beispiel eine Fluidleitung oder einen Lichtleiter, an dem Generator vorbei zu führen und / oder neben dem Generator anzuordnen. Insbesondere durch die Kombination mehrerer dieser einteiligen Ausbildungen konnte eine erhebliche Verkleinerung des Generators erzielt werden.

Durch das Vorsehen der einteilig mit der Hülse des Generators ausgebildeten Fluidleitung, welche zumindest mit ihrem von dem Generator abgewandten Ende über das Anschlussende der medizinischen, insbesondere dentalen, Vorrichtung ragt, ist in vorteilhafter Weise ein einfacher Einbau in der medizinischen, insbesondere dentalen, Vorrichtung oder ein einfacher Austausch des Generators möglich. Der Generator mit der einteilig mit der Hülse ausgebildeten Fluidleitung ist durch Schieben oder Stecken in eine Aufnahme der medizinischen, insbesondere dentalen, Vorrichtung einfügbar. Insbesondere ist die Aufnahme derart angeordnet oder bemessen, dass, wenn der dynamoelektrische Wandler in der Aufnahme aufgenommen ist und seine Betriebsposition einnimmt, die einteilig mit der Hülse des dynamoelektrischen Wandlers ausgebildete Fluidleitung zumindest mit ihrem von dem dynamoelektrischen Wandler abgewandten Ende über das Anschlussende der medizinischen, insbesondere dentalen, Vorrichtung ragt. Die einteilig mit der Hülse ausgebildete Fluidleitung, insbesondere das über das Anschlussende ragende Ende der Fluidleitung, bildet somit vorzugsweise zumindest einen Teil einer Kupplungsvorrichtung zur Verbindung mit einer Fluidquelle und / oder ein Kupplungselement zur Verbindung mit einer Fluidquelle, insbesondere ein Kupplungselement, das direkt mit einem Gegenkupplungselement, zum Beispiel mit einem Kupplungsabschnitt einer Fluidleitung, lösbar kuppelbar, insbesondere zusammensteckbar, ist.

Eine weitere Miniaturisierung des fluidbetriebenen, dynamoelektrischen Wandlers konnte vorzugsweise dadurch erreicht werden, dass die elektrische Wicklung des Stators eine gedruckte Leiterbahn aufweist. Insbesondere weist die elektrische Wicklung des Stators ein flexibles oder biegsames oder spiralförmig einrollbares Trägerelement auf, insbesondere eine flexible oder spiralförmig einrollbare Platine. Vorzugsweise ist das Trägerelement flächig oder bandförmig mit zwei einander im Wesentlichen gegenüberliegenden Seiten ausgebildet. Besonders bevorzugt ist der elektrische Leiter der elektrischen Wicklung, zum Beispiel ein Kupfer aufweisender elektrischer Leiter, auf beiden im Wesentlichen gegenüberliegenden Seiten des Trägerelements vorgesehen. Insbesondere ist zur Verbindung des elektrischen Leiters auf beiden Seiten des Trägerelements eine Durchkontaktierung des Trägerelements vorgesehen. Alternativ ist es selbstverständlich auch möglich, den elektrischen Leiter der elektrischen Wicklung nur auf einer Seite des Trägerelements vorzusehen.

Eine weitere Vereinfachung des Aufbaus und auch eine Verkleinerung des Generators wurde vorzugsweise dadurch geschaffen, dass zur Leitung des das Laufrad antreibenden Fluids im Wesentlichen nur die Hülse, die zumindest Teile des Rotors und / oder Stators umgibt, und / oder die Außenhülse und / oder die Außenhülse, die das einteilige, hülsenförmige, magnetische Rückschlusselement aufweist, vorgesehen ist. Somit entfällt in vorteilhafter Weise das Vorsehen weiterer Leitelemente für das Fluid. Zur Leitung des Fluids ist an der Hülse oder Außenhülse zum Beispiel zumindest eines der folgenden Elemente vorgesehen: Eine Bohrung, die insbesondere mit der einteilig mit der Hülse oder Außenhülse ausgebildeten Fluidleitung verbunden ist; eine an der Außenseite der Hülse oder Außenhülse vorgesehene und insbesondere mit der Bohrung und / oder mit der einteilig mit der Hülse oder Außenhülse ausgebildeten Fluidleitung verbundene erste Auslassöffnung, so dass das Fluid an die Außenseite der Hülse oder Außenhülse leitbar ist; wobei die zumindest eine an der Außenseite der Hülse oder Außenhülse, vorzugsweise ring- oder kreisbogenförmige, Nut mit der Bohrung und / oder mit der ersten Auslassöffnung verbunden ist; zumindest eine an der Außenseite der Hülse oder Außenhülse vorgesehene Einlassöffnung, so dass das Fluid von der Außenseite der Hülse oder Außenhülse, insbesondere von der Nut, in das Innere der Hülse oder Außenhülse, insbesondere zu dem Laufrad, leitbar ist; eine an der Außenseite der Hülse oder Außenhülse vorgesehene zweite Auslassöffnung, welche ausgebildet ist, das Fluid nach Passage des Laufrads an die Außenseite der Hülse zu leiten. Vorzugsweise ist / sind der Generator und / oder die medizinische, insbesondere dentale, Vorrichtung derart ausgebildet, dass das Fluid nach der Passage des Laufrads, insbesondere zur Kühlung, entlang der Außenseite der Hülse oder Außenhülse des Generators und / oder in einer an den Generator anschließenden oder diesen umgebenden Aussparung leitbar und / oder insbesondere in einer an den Generator anschließenden Fluidleitung ableitbar ist.

Gemäß einem weiteren Ausführungsbeispiel ist / sind im Inneren einer das zumindest eine Magnetelement aufnehmenden Rotorhülse und / oder im Inneren des Laufrads, insbesondere radial innerhalb der Schaufeln des Laufrads, eine Lagerstelle und / oder ein Lager, insbesondere ein Wälz- oder Kugellager, zur drehbaren Lagerung der Rotorhülse und / oder des Laufrads angeordnet. Dies bewirkt eine, insbesondere axiale Verkürzung und einen noch kompakteren Aufbau des Generators. Vorzugsweise ist auch eine im Inneren der Rotorhülse und / oder im Inneren des Laufrads, insbesondere radial innerhalb der Schaufeln des Laufrads, aufgenommene Rotorwelle zur drehbaren Lagerung der Rotorhülse vorgesehen.

Gemäß einem anderen Ausführungsbeispiel weist die einteilig mit dem Laufrad ausgebildete Rotorhülse einen ersten Abschnitt, in dem zumindest ein Teil des zumindest einen Magnetelements aufgenommen ist, und einen zweiten Abschnitt auf, an dem die Schaufeln des Laufrads vorgesehen sind, wobei der Außendurchmesser des ersten Abschnitts geringer ist als der Außendurchmesser des zweiten Abschnitts, so dass an dem ersten Abschnitt ein Rücksprung gebildet ist. Vorzugsweise ist in oder an dem Rücksprung zumindest ein Teil der elektrischen Wicklung und / oder zumindest ein Teil eines Träger- oder Lagerelements für die elektrische Wicklung und / oder ein Luftspalt des Generators vorgesehen. Auch durch dieses Ausführungsbeispiel wird die Kompaktheit des Generators erhöht und seine Abmessung, insbesondere sein Durchmesser, verringert.

Es wird ausdrücklich darauf hingewiesen, dass eine Kombination von zwei oder mehr der im Vorstehenden beschriebenen Ausführungsbeispiel nicht nur möglich ist, sondern dass dadurch besondere Vorteile erzielbar sind, insbesondere eine zusätzliche Miniaturisierung des fluidbetriebenen, dynamoelektrischen Wandlers.

Die medizinische, insbesondere dentale, Vorrichtung, in welcher der fluidbetriebene, dynamoelektrische Wandler vorgesehen ist, umfasst zum Beispiel ein gerades, gebogenes oder pistolenförmiges Handstück oder Handgriffelement, einen Adapter oder eine Kupplungsvorrichtung. Das Handstück oder Handgriffelement, der Adapter oder die Kupplungsvorrichtung weisen zum Beispiel einen elektrischen Verbraucher auf, vorzugsweise eine Beleuchtungsvorrichtung, insbesondere zumindest ein optisches Halbleiterelement, der mit der von dem Generator erzeugten elektrischen Energie versorgbar und / oder betreibbar ist. Vorzugsweise sind der Generator und der elektrische Verbraucher voneinander trennbar, insbesondere sind sie nicht in demselben Bauteil angeordnet. Vorzugsweise umfassen das Handstück oder Handgriffelement, der Adapter oder die Kupplungsvorrichtung elektrische Kontakte und / oder elektrische Leiter auf, welche den Generator mit dem elektrischen Verbraucher verbinden. Das Handstück oder Handgriffelement umfasst insbesondere zumindest ein fluidbetriebenes Antriebselement zum in Bewegung versetzen eines mit dem Handstück oder Handgriffelement verbindbaren Werkzeugs. Der Adapter oder die Kupplungsvorrichtung umfassen insbesondere ein lösbar mit einer Fluidquelle verbindbares Anschlussende und ein davon beabstandetes Verbindungsende zur lösbaren Verbindung mit einem medizinischen, insbesondere dentalen, Instrument, zum Beispiel einem Handgriffelement.

Die Erfindung wird nachfolgend anhand bevorzugter Ausführungsbeispiele und Bezug nehmend auf die beigefügten Zeichnungen erläutert. Es zeigt die
Figur 1 eine Explosionsdarstellung eines Ausführungsbeispiels eines fluidbetriebenen, dynamoelektrischen Wandlers.
Figur 2 einen Längsschnitt durch den fluidbetriebenen, dynamoelektrischen Wandler der Figur 1.
Figur 3 einen Längsschnitt durch eine medizinische, insbesondere dentale, Vorrichtung in Form einer Kupplungsvorrichtung oder eines Adapters mit dem fluidbetriebenen, dynamoelektrischen Wandler der Figuren 1 und 2.
Figur 4 eine medizinische, insbesondere dentale, Vorrichtung in Form eines Handstücks mit dem fluidbetriebenen, dynamoelektrischen Wandler.

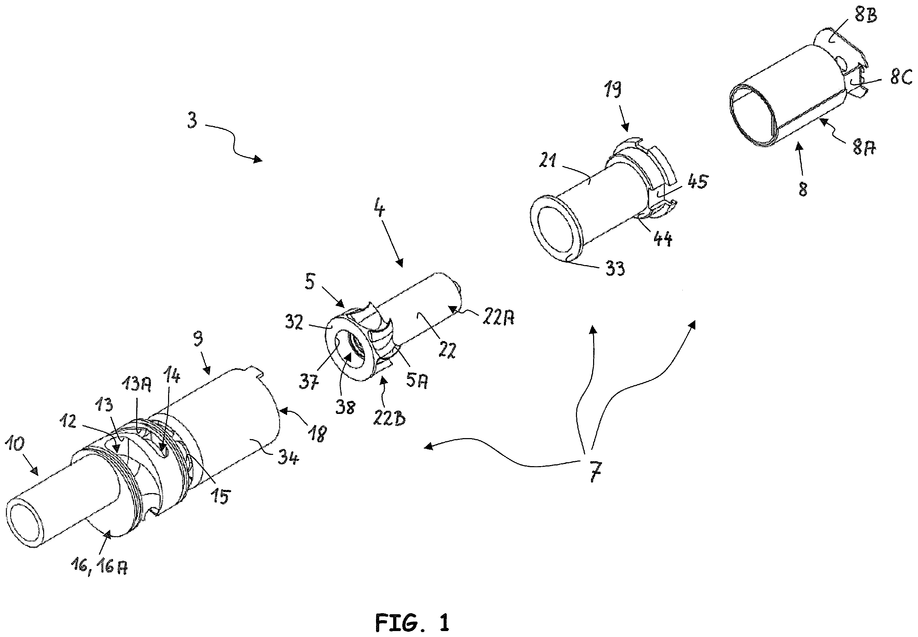

Die Figur 1 zeigt als Hauptelemente des fluidbetriebenen, dynamoelektrischen Wandlers 3 einen Rotor 4 und einen Stator 7. Der Rotor 4 umfasst ein durch ein Fluid in Drehung versetzbares Laufrad 5 und zumindest ein Magnetelement 6. Das Magnetelement 6 ist mit dem Laufrad 5 verbunden, so dass das Magnetelement 6 durch das in Drehung versetzbare Laufrad 5 ebenfalls in Drehung versetzbar ist. Der Stator 7 weist eine elektrische Wicklung 8 auf, die mit dem in Drehung versetzbaren Magnetelement 6 derart zusammenwirkt, dass in der elektrischen Wicklung 8 elektrische Energie erzeugbar ist.

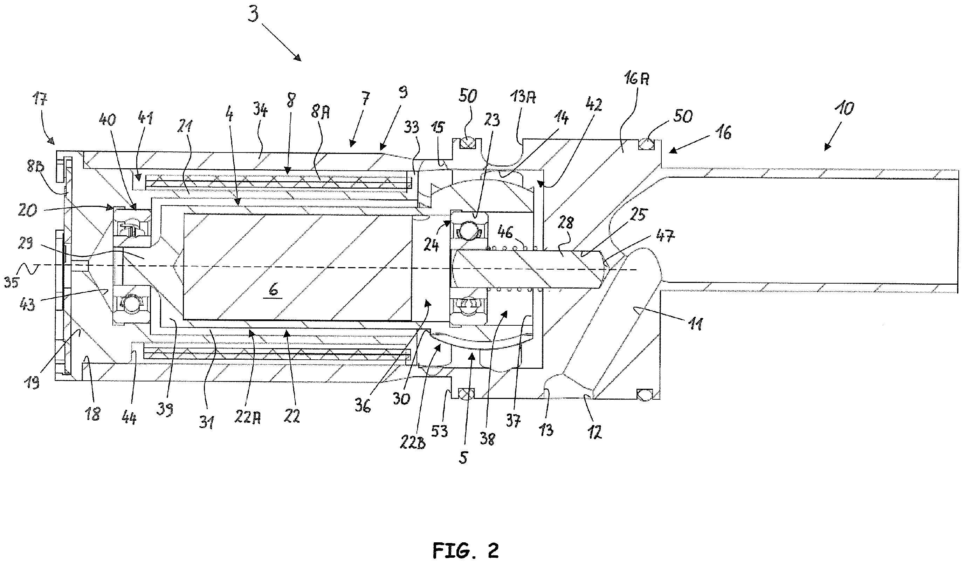

Im Folgenden wird unter Bezugnahme auf die Figuren 1 und 2 der fluidbetriebene, dynamoelektrische Wandler 3 (Generator) im Detail beschrieben.

Der Rotor 4 weist eine Längs- oder Drehachse 35 auf, um welche der Rotor 4 drehbar angeordnet ist. Das Magnetelement 6 des Rotors 4 ist vorzugsweise als Permanentmagnet ausgebildet. Das Magnetelement 6 ist als zweipoliges oder mehrpoliges Magnetelement ausgebildet. Das Magnetelement 6 ist vorzugsweise zylindrisch geformt.

Das Magnetelement 6 ist vorzugsweise in einer Rotorhülse 22 angeordnet oder aufgenommen und/ oder darin befestigt, insbesondere verklebt oder verpresst. Die Rotorhülse 22 ist vorzugsweise hohl ausgebildet und weist insbesondere einen Innenraum 36 auf. Vorzugsweise ist in dem Innenraum 36 zumindest das Magnetelement 6 aufgenommen. Vorzugsweise ist die Rotorhülse 22, insbesondere der Innenraum 36, zylindrisch und / oder länglich geformt. Vorzugsweise ist ein Ende der Rotorhülse 22 durch eine Endwand 39 verschlossen.

Das mit Fluid antreibbare Laufrad 5 des Rotors 4 weist vorzugsweise mehrere Schaufeln 5A auf. Das Laufrad 5 ist vorzugsweise im Wesentlichen konzentrisch mit dem Magnetelement 6 und / oder mit der Rotorhülse 22 angeordnet. Das Laufrad 5, insbesondere die Schaufeln 5A, ist / sind vorzugsweise radial um die Drehachse 35 angeordnet. Das Laufrad 5 ist vorzugsweise hohl ausgebildet und / oder weist einen Hohlraum 38 auf. Besonders bevorzugt ist das Laufrad 5, insbesondere der Hohlraum 38, zur Aufnahme zumindest eines Bauteils des Generators 3 ausgebildet, zum Beispiel zur Aufnahme eines Lagers 24 zur drehbaren Lagerung des Laufrads 5 und / oder zur Aufnahme einer Welle oder Rotorwelle 28 zur drehbaren Lagerung des Laufrads 5. Vorzugsweise ist in dem Laufrad 5, insbesondere in dem Hohlraum 38, eine Lagerstelle 23 vorgesehen, insbesondere für das Lager 24 zur drehbaren Lagerung des Laufrads 5. Vorzugsweise umfasst das Lager 24 ein Wälz- oder Kugellager. Vorzugsweise sind die Schaufeln 5A des Laufrads 5 radial um den Hohlraum 38 angeordnet. Vorzugsweise weist der Hohlraum 38 eine Innenwand auf, von welcher sich die Schaufeln 5A radial nach außen erstrecken.

Vorzugsweise ist ein Ringflansch 32 an zumindest einem Ende des Laufrads 5 vorgesehen, der das Laufrad 5 an seinem Außenumfang umläuft. Vorzugsweise schließen die Schaufeln 5A des Laufrads 5 an den Ringflansch 32 an oder sind mit dem Ringflansch 32 verbunden. Vorzugsweise bildet der Ringflansch 32 ein axiales Ende des Laufrads 5. Vorzugsweise ist der Ringflansch 32 vorgesehen, das Fluid, welches das Laufrad 5 antreibt oder mit welchem das Laufrad 5 beaufschlagbar ist, zu lenken. Vorzugsweise ist der Ringflansch 32 einteilig mit dem Laufrad 5 und / oder den Schaufeln 5A ausgebildet.

Besonders bevorzugt ist das Laufrad 5, insbesondere sind die Schaufeln 5A des Laufrads 5, einteilig mit der Rotorhülse 22 ausgebildet. Besonders bevorzugt ist das Laufrad 5, insbesondere sind die Schaufeln 5A, an der Rotorhülse 22 vorgesehen. Insbesondere sind die Schaufeln 5A des Laufrads 5 an einer Außenseite der Rotorhülse 22 und / oder an einem Ende der Rotorhülse 22 angeordnet. Insbesondere sind das Laufrad 5, insbesondere die Schaufeln 5A, und die Rotorhülse 22 somit als ein einziges, gemeinsames Bauteil ausgebildet. Vorzugsweise sind der Innenraum 36 der Rotorhülse 22 und der Hohlraum 38 des Laufrads miteinander verbunden oder bilden einen gemeinsamen Innenraum.

Bevorzugt ist der Innenraum 36 der Rotorhülse 22 oder der aus dem Innenraum 36 und dem Hohlraum 38 gebildete, gemeinsame Innenraum zur Aufnahme eines Lagers 24 zur drehbaren Lagerung des Laufrads 5 und der Rotorhülse 22 mit dem Magnetelement 6 und / oder zur Aufnahme einer Rotorwelle 28 zur drehbaren Lagerung des Laufrads 5 und der Rotorhülse 22 mit dem Magnetelement 6 ausgebildet. Vorzugsweise ist in dem Innenraum 36 oder dem gemeinsamen Innenraum eine Lagerstelle 23 vorgesehen, insbesondere für das Lager 24. Vorzugsweise umfasst das Lager 24 ein Wälz- oder Kugellager. Vorzugsweise sind die Schaufeln 5A des Laufrads 5 radial um den Innenraum 36 oder den gemeinsamen Innenraum angeordnet. Vorzugsweise weist der gemeinsame Innenraum eine Innenwand auf, von welcher sich die Schaufeln 5A radial nach außen erstrecken.

Vorzugsweise ist an der Rotorhülse 22, insbesondere an einem Ende der Rotorhülse 22, oder an dem Laufrad 5 eine Öffnung 37 vorgesehen, durch welche zumindest das Magnetelement 6, gegebenenfalls weitere Bauteil des Generators 3, insbesondere das Lager 24 und / oder die Welle 28, in die Rotorhülse 22 und / oder in das Laufrad 5 einfügbar sind. Vorzugsweise ist die Öffnung 37 an dem der Endwand 39 gegenüberliegenden Ende der Rotorhülse 22 und / oder des Laufrads 5 angeordnet. Vorzugsweise ist die Rotorhülse 22 mit dem einteilig ausgebildeten Laufrad 5 als, insbesondere topfförmige, Hülse mit einer Bohrung 36, 38 ausgebildet, in welcher das Magnetelement 6 und insbesondere das Lager 24 und / oder die Welle 28 angeordnet sind.

Vorzugsweise ist ein insbesondere einteilig mit der Rotorhülse 22 und / oder der Endwand 39 ausgebildeter Rotorzapfen 29 zur drehbaren Lagerung der Rotorhülse 22 und / oder des Laufrads 5 vorgesehen. Vorzugsweise ist der Rotorzapfen 29 an einem Ende der Rotorhülse 22 angeordnet, insbesondere an der Endwand 39 der Rotorhülse 22. Vorzugsweise ist an dem Rotorzapfen 29 ein Lager 40, insbesondere ein Wälz- oder Kugellager, zur drehbaren Lagerung der Rotorhülse 22 und / oder des Laufrads 5 vorgesehen.

Vorzugsweise weist die Rotorhülse 22 einen ersten Abschnitt 22A, in dem zumindest ein Teil des zumindest einen Magnetelements 6 aufgenommen ist, und einen zweiten Abschnitt 22B auf, an dem das Laufrad 5 oder die Schaufeln 5A des Laufrads 5 vorgesehen ist / sind. Der Außendurchmesser des ersten Abschnitts 22A ist geringer als der Außendurchmesser des zweiten Abschnitts 22B. Dadurch ist an dem ersten Abschnitt 22A ein Rücksprung 30 gebildet. Die Seitenwand oder die axiale Begrenzung des Rücksprungs 30 ist insbesondere durch die Schaufeln 5A des Laufrads 5, insbesondere durch deren seitliche Enden oder freie Seitenenden, gebildet. Vorzugsweise schließt das Laufrad 5, insbesondere schließen die Schaufeln 5A des Laufrads 5, an den ersten Abschnitt 22A an. Vorzugsweise ist der Außendurchmesser des Laufrads 5 oder der Schaufeln 5A, zumindest der äußeren oder radialen Enden der Schaufeln 5A, größer als der Außendurchmesser des ersten Abschnitts 22A.

Vorzugsweise ist in oder an dem Rücksprung 30 zumindest ein Teil der elektrischen Wicklung 8 und / oder zumindest ein Teil eines Lager- oder Trägerelements 21 für die elektrische Wicklung 8 und / oder eines Luftspalts 31 des dynamoelektrischen Wandlers 3 vorgesehen. Die elektrische Wicklung 8 und / oder zumindest ein Teil des Lager- oder Trägerelements 21 und / oder der Luftspalt 31 ist / sind somit vorzugsweise axial an das Laufrad 5, insbesondere an die Schaufeln 5A des Laufrads 5, anschließend angeordnet. Die elektrische Wicklung 8 und / oder zumindest ein Teil des Lager- oder Trägerelements 21 und / oder der Luftspalt 31 erstrecken sich vorzugsweise über im Wesentlichen den gesamten ersten Abschnitt 22A.

Die elektrische Wicklung 8 des Stators 7 ist vorzugsweise zumindest um einen Teil des Rotors 4, insbesondere um das Magnetelement 6 und / oder die Rotorhülse 22, vorzugsweise deren Abschnitt 22A, angeordnet. Die elektrische Wicklung 8 umfasst zum Beispiel zumindest eine gewickelte Spulenwicklung. Bevorzugt umfasst die elektrische Wicklung 8 zumindest eine gedruckte Leiterbahn 8A. Insbesondere weist die elektrische Wicklung 8 ein flexibles oder biegsames oder spiralförmig einrollbares Trägerelement auf, insbesondere eine flexible oder spiralförmig einrollbare Platine, auf dem / der die gedruckte Leiterbahn 8A angeordnet ist. Wie aus der Schnittdarstellung der Figur 2 zu erkennen ist, weist die elektrische Wicklung 8 aufgrund der spiralförmigen Anordnung des spiralförmig einrollbaren Trägerelements vorzugsweise 2 - 4 (radial) übereinander angeordnete Ebenen oder Schichten auf. Das spiralförmig einrollbare Trägerelement ist vorzugsweise beidseitig mit gedruckten Leiterbahnen 8A versehen.

Die elektrische Wicklung 8 des Stators 7 ist entweder frei, ohne zusätzliches Träger- oder Lagerelement, um den Rotor 4 angeordnet oder es ist ein Träger- oder Lagerelement 21 für die elektrische Wicklung 8 vorgesehen. Das Träger- oder Lagerelement 21 ist vorzugsweise zylindrisch, insbesondere hohlzylindrisch oder hülsenförmig, ausgebildet. Das Träger- oder Lagerelement 21 weist vorzugsweise eine in etwa gleiche oder etwas längere axiale Abmessung als die elektrische Wicklung 8 auf. Das Träger- oder Lagerelement 21 umgibt vorzugsweise zumindest Teile des Rotors 4 oder nimmt zumindest Teile des Rotors 4 in seinem Inneren auf, zum Beispiel das Magnetelement 6 und / oder die Rotorhülse 22. Das Träger- oder Lagerelement 21 ist insbesondere durch den Luftspalt 31 von dem Magnetelement 6 und / oder der Rotorhülse 22 getrennt.

Das Träger- oder Lagerelement 21 weist vorzugsweise zumindest ein freies Ende auf, welches insbesondere dem Laufrad 5 zugewandt ist oder an das Laufrad 5, bevorzugt an die Schaufeln 5A, anschließt, jedoch insbesondere davon beabstandet ist. Vorzugsweise weist dieses freie Ende einen Ringflansch 33 auf, der anschließend an das Laufrad 5, insbesondere an die Schaufeln 5A, angeordnet ist. Vorzugsweise entspricht die (radiale) Höhe des Ringflansches 33 in etwa der (radialen) Höhe der elektrischen Wicklung 8. Vorzugsweise bilden das Träger- oder Lagerelement 21 und der Ringflansch 33, insbesondere mit weiteren Bauteilen des Generators 3, zum Beispiel mit einem Verschlusselement 19 und / oder einer Hülse 9, eine Kammer 41 für die elektrische Wicklung 8. Vorzugsweise ist die Kammer 41 derart ausgebildet, dass sie die elektrische Wicklung 8 von weiteren Bauteil des Generators 3 abgrenzt oder abschließt, insbesondere vor einer direkten Beaufschlagung mit dem das Laufrad 5 antreibenden Fluid schützt.

Vorzugsweise ist der Ringflansch 33 auch als Leitelement für das das Laufrad 5 antreibenden Fluid ausgebildet. Insbesondere bildet der Ringflansch 33 mit dem Ringflansch 32 eine Leitvorrichtung für das Fluid, zum Beispiel zum Leiten des Fluids zu dem Laufrad 5 oder von dem Laufrad 5 weg. Vorzugsweise wirken die beiden Ringflansche 32, 33 hierzu mit weiteren Bauteil des Generators 3 zusammen, zum Beispiel mit der Hülse 9 und / oder mit einer zur Fluidleitung vorgesehenen Öffnung 12, 14, 15, Bohrung 11 oder Nut 13 des Generators 3. Vorzugsweise ist der Ringflansch 33 vorgesehen, insbesondere mit weiteren Bauteilen des Generators 3, zum Beispiel der Hülse 9, eine Kammer 42 für das Laufrad 5 zu bilden. Vorzugsweise trennt der Ringflansch 33 die Kammer 41 für die elektrische Wicklung 8 von der Kammer 42 für das Laufrad 5.

Vorzugsweise ist das Träger- oder Lagerelement 21 mit einem Verschlusselement 19 verbunden, insbesondere einteilig ausgebildet. Das Verschlusselement 19 ist insbesondere dazu vorgesehen, eine Öffnung 18 des Generators 3, zum Beispiel einer Außenhülse 9 des Generators 3, zu verschließen, vorzugsweise lösbar zu verschließen. Das Verschlusselement 19 ist vorzugsweise ausgebildet, zumindest teilweise in die Öffnung 18 eingefügt zu werden. Das Verschlusselement 19 ist vorzugsweise vorgesehen, mit zumindest einer Seite oder Fläche eine Außenwand oder Außenfläche des Generators 3 zu bilden. Das Verschlusselement 19 ist vorzugsweise an einem Ende des Träger- oder Lagerelements 21 vorgesehen und schließt insbesondere ein Ende des Träger- oder Lagerelements 21 ab. Das Verschlusselement 19 umfasst vorzugsweise zumindest eine Aufnahme oder Bohrung 43 zur Aufnahme eines Teils des Rotors 4, insbesondere der Rotorhülse 22 und / oder des Rotorzapfens 29, oder des Lagers 40 zur drehbaren Lagerung der Rotorhülse 22 und / oder des Laufrads 5. Das Verschlusselement 19, insbesondere die Bohrung 43, umfasst vorzugsweise eine Lagerstelle 20 zur drehbaren Lagerung des Magnetelements 6 des Rotors 4 und / oder des Laufrads 5. An der Lagerstelle 20 ist insbesondere das Lager 40 angeordnet.

Vorzugsweise ist der Außendurchmesser des Träger- oder Lagerelements 21 geringer als der Außendurchmesser des Verschlusselements 19, so dass insbesondere ein Rücksprung 44 gebildet ist. Vorzugsweise begrenzt das Verschlusselement 19, insbesondere der Rücksprung 44, die Kammer 41. Vorzugsweise ist die elektrische Wicklung 8 zwischen dem Rücksprung 44 und dem Ringflansch 33 angeordnet.

Vorzugsweise umfasst der dynamoelektrische Wandler 3 eine Hülse oder Außenhülse 9, die zumindest Teile des Rotors 4 und / oder des Stators 7, insbesondere zumindest die elektrische Wicklung 8, umgibt. Die Hülse 9 umfasst vorzugsweise zumindest einen zylindrischen Abschnitt, in welchem insbesondere das zumindest eine Magnetelement 6 und / oder die Rotorhülse 22 und / oder die elektrische Wicklung 8 angeordnet ist / sind. Vorzugsweise ist die Hülse oder Außenhülse 9 einteilig und / oder länglich ausgebildet. Vorzugsweise umgibt die Hülse 9 den Rotor 4 und die elektrische Wicklung 8 des Stators 7 entlang ihrer gesamten axialen Ausdehnung. Vorzugsweise ist der Generator 3 als austauschbare Generatorpatrone ausgebildet, wobei die Außenhülse 9 insbesondere die Patronenhülse bildet, an der die Bauteile des Generators 3 direkt oder indirekt gelagert und / oder befestigt sind.

Vorzugsweise weist die Hülse oder Außenhülse 9 ein Aufnahmeende 17 auf, an dem die Öffnung 18 vorgesehen ist, durch die der Rotor 4 und die elektrische Wicklung 8 des Stators 7 einfügbar sind. Die Öffnung 18 ist insbesondere durch das im Vorstehenden beschriebene Verschlusselement 19 lösbar verschließbar. Die Hülse oder Außenhülse 9 und das Verschlusselement 19 begrenzen somit vorzugsweise eine Kammer, in welcher der Rotor 4 und die elektrische Wicklung 8 aufnehmbar oder aufgenommen sind.

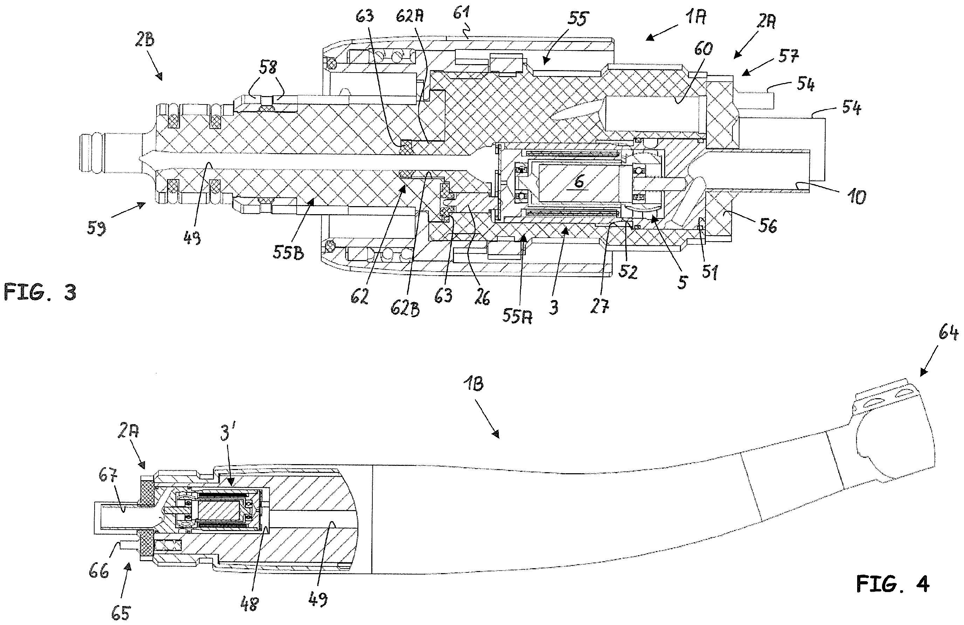

Die im Vorstehenden beschriebene gedruckte Leiterbahn 8A weist vorzugsweise einen Abschnitt 8B auf, der an der Außenseite der Hülse 9 des dynamoelektrischen Wandlers 3 und / oder an der Außenseite des Verschlusselements 19 vorgesehen ist, so dass der Abschnitt 8B insbesondere mit einem außerhalb der Hülse 9 angeordneten elektrischen Kontakt 26 verbindbar oder verbunden ist (siehe auch Figur 3). Bevorzugt ist dafür eine Öffnung und / oder ein Durchlass 45 für die gedruckte Leiterbahn 8A oder den Abschnitt 8B oder einen Verbindungsabschnitt 8C der gedruckten Leiterbahn 8A an der Hülse 9 und / oder an dem Verschlusselement 19 vorgesehen. Vorzugsweise verbindet der Durchlass 45 die Kammer 41 für die elektrische Wicklung 8 mit der Außenseite der Hülse 9 und / oder des Verschlusselements 19. Vorzugsweise erstreckt sich die gedruckte Leiterbahn 8A oder der Verbindungsabschnitt 8C durch den Durchlass 45, so dass der Abschnitt 8B mit dem im Inneren der Hülse 9 angeordneten Teil der gedruckte Leiterbahn 8A verbunden ist.

Vorzugsweise ist der Abschnitt 8B der gedruckten Leiterbahn 8A im Wesentlichen parallel zur Außenseite des Verschlusselements 19 angeordnet. Vorzugsweise ist der Abschnitt 8B der gedruckten Leiterbahn 8A im Wesentlichen rechtwinkelig zur Längsachse 35 des Rotors 4 und / oder im Wesentlichen konzentrisch mit dem Rotor 4 angeordnet. Vorzugsweise ist zumindest ein Teil des Verschlusselements 19 zwischen dem Abschnitt 8B und dem im Inneren der Hülse 9 angeordneten Teil der gedruckte Leiterbahn 8A angeordnet. Vorzugsweise sind der Abschnitt 8B und / oder der Verbindungsabschnitt 8C der gedruckten Leiterbahn 8A im Wesentlichen flächig ausgebildet (im Gegensatz zur der spiraligen Anordnung der übrigen gedruckten Leiterbahn 8A). Vorzugsweise sind an einer Seite des flächigen Abschnitts 8B, insbesondere an der vom Rotor 4 abgewandten Seite, zwei frei liegende, elektrische Kontakte vorgesehen.

Vorzugsweise weist die Hülse oder Außenhülse 9 ein anschlussseitiges Ende 16 auf, das insbesondere dem Aufnahmeende 17 im Wesentlichen gegenüberliegt. Das anschlussseitige Ende 16 ist insbesondere durch eine anschlussseitige Endwand 16A gebildet und / oder umfasst eine anschlussseitige Endwand 16A. Das anschlussseitige Ende 16 und / oder die anschlussseitige Endwand 16A ist / sind insbesondere einteilig mit der Hülse oder Außenhülse 9 ausgebildet. Die Hülse oder Außenhülse 9 und die anschlussseitige Endwand 16A begrenzen somit vorzugsweise eine Kammer, insbesondere gemeinsam mit dem Verschlusselement 19, in welcher der Rotor 4 und die elektrische Wicklung 8 aufnehmbar oder aufgenommen sind.

Die im Vorstehenden beschriebene Rotorwelle 28 zur drehbaren Lagerung des Rotors 4 und / oder des Laufrads 5 ist vorzugsweise in einer außerhalb der Rotorhülse 22 und / oder des Laufrads 5 vorgesehenen Lagerstelle 25 gelagert, insbesondere in einer Wand der Hülse 9. Besonders bevorzugt ist die Lagerstelle 25 zur drehbaren Lagerung des Laufrads 5 und / oder des Rotors 4 in der anschlussseitigen Endwand 16A der Hülse 9 vorgesehen. Die Lagerstelle 25 umfasst insbesondere eine Bohrung 47 in der anschlussseitigen Endwand 16A zur Aufnahme zumindest eines Teils der Rotorwelle 28. Vorzugsweise ist die Rotorwelle 28 drehfest in der anschlussseitigen Endwand 16A und / oder in deren Bohrung 47 aufgenommen. Vorzugsweise ist an der Rotorwelle 28 ein Federelement 46 vorgesehen, das den Rotor 4 vorspannt. Vorzugsweise ist das Federelement 46 an der anschlussseitigen Endwand 16A gelagert.

Vorzugsweise ist an oder in der Hülse oder Außenhülse 9 zumindest ein Element vorgesehen, das zur Leitung des das Laufrad 5 antreibenden Fluids ausgebildet ist. Dieses Leitelement umfasst zum Beispiel zumindest eines der im Folgenden beschriebenen Elemente:
- zumindest eine Einlassöffnung 14, die ausgebildet ist, ein Fluid zum Antrieb des Laufrads 5 von der Außenseite der Hülse oder Außenhülse 9 in das Innere der Hülse oder Außenhülse 9, insbesondere zu dem Laufrad 5, zu leiten. Vorzugsweise ist die zumindest eine Einlassöffnung 14 radial zu dem Laufrad 5 angeordnet. Vorzugsweise ist die zumindest eine Einlassöffnung 14 derart angeordnet, dass das Laufrad 5 radial mit dem Fluid beaufschlagbar ist. Vorzugsweise umfasst die zumindest eines Einlassöffnung 14 eine Durchbohrung der Hülse oder Außenhülse 9. Vorzugsweise sind mehrere Einlassöffnungen 14 vorgesehen, die im Wesentlichen in regelmäßigen Abständen, insbesondere Winkelbögen, zueinander angeordnet sind. Vorzugsweise ist die zumindest eine Einlassöffnung 14 mit der Kammer 42 für das Laufrad 5 fluidleitend verbunden. Vorzugsweise ist die Hülse oder Außenhülse 9 derart ausgebildet oder derart in einer medizinischen, insbesondere dentalen, Vorrichtung 1A, 1B angeordnet, dass das Fluid an der Außenseite der Hülse oder Außenhülse 9 leitbar ist, insbesondere zu der zumindest einen Einlassöffnung 14 leitbar ist. Vorzugsweise ist der Generator 3 in einer Aufnahme 27, 48 einer medizinischen, insbesondere dentalen, Vorrichtung 1A, 1B (siehe Figuren 3 und 4) derart angeordnet, dass das Fluid zum Antrieb des Laufrads 5 in der Aufnahme 27, 48 oder zwischen einer Wand der Aufnahme 27, 48 und der Hülse oder Außenhülse 9 des Generators 3 leitbar ist;

- zumindest eine, vorzugsweise ring- oder kreisbogenförmige, Nut 13 an der Außenseite der Außenhülse 9, die mit der zumindest einen Einlassöffnung 14 verbunden ist, so dass ein Fluid zum Antrieb des Laufrads 5 über die Nut 13 zu der Einlassöffnung 14 leitbar ist. Vorzugsweise ist eine Nut 13 vorgesehen, die im Wesentlichen parallel zur anschlussseitigen Endwand 16A verläuft. Vorzugsweise ist zumindest ein von der Nut 13 abzweigende Nut 13A vorgesehen, welche die zumindest eine Einlassöffnung 14 mit der kreisbogenförmigen Nut 13 verbindet. Vorzugsweise ist jeder Einlassöffnung 14 eine abzweigende Nut 13A zugeordnet. Vorzugsweise schließt die zumindest eine abzweigende Nut 13A einen Winkel mit der anschlussseitigen Endwand 16A ein. Vorzugsweise sind mehrere abzweigende Nut 13A vorgesehen, die im Wesentlichen in regelmäßigen Abständen von der kreisbogenförmigen Nut 13 abzweigen;
- eine einteilig mit der Hülse oder Außenhülse 9 ausgebildete Fluidleitung 10, die über ein Ende, insbesondere das anschlussseitige Ende 16, der Außenhülse 9 ragt und die mit der Einlassöffnung 14 und / oder der Nut 13 fluidleitend verbunden ist, so dass ein in der Fluidleitung 10 fließendes Fluid zu der Einlassöffnung 14 und / oder der Nut 13 leitbar ist. Die einteilig mit der Hülse oder Außenhülse 9 ausgebildete Fluidleitung 10 steht insbesondere von der Hülse oder Außenhülse 9 ab. Die einteilig mit der Hülse oder Außenhülse 9 ausgebildete Fluidleitung 10 ist insbesondere an einem anschlussseitigen Ende 16 oder an einer anschlussseitigen Endwand 16A der Hülse oder Außenhülse 9 angeordnet. Vorzugsweise ist die Fluidleitung 10 rohrförmig oder hohlzylindrisch geformt. Vorzugsweise ist die Fluidleitung 10 außermittig oder versetzt zur Längsachse 35 angeordnet. Vorzugsweise ist der Außendurchmesser der einteilig mit der Hülse oder Außenhülse 9 ausgebildeten Fluidleitung 10 geringer ist als der Außendurchmesser zumindest eines Abschnitts der Hülse 9, insbesondere des anschlussseitigen Endes 16 oder der anschlussseitigen Endwand 16A;
- zumindest ein Bohrung 11 in der Hülse oder Außenhülse 9, welche die einteilig mit der Hülse oder Außenhülse 9 ausgebildete Fluidleitung 10 mit der Einlassöffnung 14 und / oder der Nut 13 fluidleitend verbindet. Die Bohrung 11 ist vorzugsweise in der anschlussseitigen Endwand 16A vorgesehen. Der Innendurchmesser der Bohrung 11 ist vorzugsweise geringer als der Innendurchmesser der Fluidleitung 10. Die Bohrung 11 ist vorzugsweise gewinkelt zur Fluidleitung 10 angeordnet, wobei der Winkel insbesondere weniger als 90° beträgt, besonders bevorzugt zwischen etwa 45° und 85° liegt. Die Bohrung 11 mündet vorzugsweise mit einem Ende in die Fluidleitung 10. Die Bohrung 11 endet vorzugsweise an der Außenseite der Hülse oder Außenhülse 9;
- eine, insbesondere an der Außenseite der Hülse oder Außenhülse 9 vorgesehene, mit der Bohrung 11 und / oder der Fluidleitung 10 verbundene erste Auslassöffnung 12, so dass das Fluid an die Außenseite der Hülse oder Außenhülse 9 und / oder in die Nut 13 und / oder zur Einlassöffnung 14 und / oder in die Aufnahme 27, 48 der medizinischen, insbesondere dentalen, Vorrichtung 1A, 1B leitbar ist. Vorzugsweise ist die erste Auslassöffnung 12 in oder an der Nut 13 vorgesehen;
- zumindest eine zweite Auslassöffnung 15, welche ausgebildet ist, das Fluid nach Passage des Laufrads 5 an die Außenseite der Außenhülse 9 zu leiten. Vorzugsweise ist die zweite Auslassöffnung 15 mit der Kammer 42 für das Laufrad 5 fluidleitend verbunden. Vorzugsweise ist die Hülse oder Außenhülse 9 derart ausgebildet oder derart in einer medizinischen, insbesondere dentalen, Vorrichtung 1A, 1B angeordnet, dass das Fluid nach der Passage des Laufrads 5 an der Außenseite der Hülse oder Außenhülse 9 leitbar ist, insbesondere in Richtung einer an den Generator 3 und / oder die Aufnahme 27, 48 anschließenden Fluidleitung 49 leitbar ist (siehe Figuren 3 und 4). Vorzugsweise ist der Generator 3 in der Aufnahme 27, 48 derart angeordnet, dass das Fluid nach der Passage des Laufrads 5 in der Aufnahme 27, 48 oder zwischen einer Wand der Aufnahme 27, 48 und der Hülse oder Außenhülse 9 des Generators 3 leitbar ist. Vorzugsweise ist die zumindest eine zweite Auslassöffnung 15 anschließend an den Ringflansch 33 und / oder axial zwischen dem Laufrad 5 und der elektrischen Wicklung 8 angeordnet.

Vorzugsweise sind zumindest die Bohrung 11, die Nut 13, 13A und die Öffnungen 12, 14, 15 in einem Abschnitt der Hülse oder Außenhülse 9, insbesondere in einem Abschnitt mit der anschlussseitigen Endwand 16A, vorgesehen, der anschließend an einen Abschnitt 34 der Hülse oder Außenhülse 9 angeordnet ist, der das Magnetelement 6 und / oder die elektrische Wicklung 8 umgibt. Vorzugsweise ist an der Außenseite der Hülse oder Außenhülse 9 zumindest ein Dichtelement 50 vorgesehen, das zum Beispiel zumindest zwei der im Vorstehenden beschriebenen Elemente zur Leitung des das Laufrad 5 antreibenden Fluids im Wesentlichen fluiddicht voneinander trennt, vorzugsweise die zumindest eine zweite Auslassöffnung 15 von zumindest einem der anderen Elemente 11, 12, 13, 13A, 14 fluiddicht trennt. Vorzugsweise ist an der Außenseite der Hülse oder Außenhülse 9 zumindest ein Dichtelement 50 vorgesehen, das jenen Abschnitt der Außenseite der Hülse oder Außenhülse 9, an dem das Fluid leitbar ist, abdichtet.

Vorzugsweise umfasst der Generator ein magnetischen Rückschlusselement zum Konzentrieren und Lenken der magnetischen Feldlinien des zumindest einen Magnetelements 6 durch die elektrische Wicklung 8. Bevorzugt umfasst das Rückschlusselement ein einteiliges, hülsenförmiges, magnetisches Rückschlusselement 34, das den Rotor 4 und die elektrische Wicklung 8 umgibt und das ein magnetisch leitendes Material aufweist. Besonders bevorzugt ist das einteilige, hülsenförmige, magnetische Rückschlusselement 34 Teil der, insbesondere einteiligen, Hülse oder Außenhülse 9 des dynamoelektrischen Wandlers 3. Das einteilige, hülsenförmige, magnetische Rückschlusselement 34 ist insbesondere einteilig mit der Hülse oder Außenhülse 9 verbunden oder ausgebildet und / oder das einteilige, hülsenförmige, magnetische Rückschlusselement 34 ist Teil der Patronenhülse des Generators 3. Das einteilige, hülsenförmige, magnetische Rückschlusselement 34 ist vorzugsweise aus Stahl gebildet. Das einteilige, hülsenförmige, magnetische Rückschlusselement 34 weist vorzugsweise eine Wandstärke von etwa 0,2 mm bis etwa 1,0 mm, insbesondere von etwa 0,4 mm bis etwa 0,5 mm, auf.

Der vorstehenden Beschreibung entsprechend ist vorzugsweise die Öffnung 18, durch die der Rotor 4 und die elektrische Wicklung 8 des Stators 7 einfügbar sind, an dem einteiligen, hülsenförmigen, magnetischen Rückschlusselement 34, insbesondere an dessen Aufnahmeende 17, vorgesehen. Im Übrigen kann die Öffnung 18 an dem einteiligen, hülsenförmigen, magnetischen Rückschlusselement 34 die gleichen Merkmale aufweisen, wie sie im Vorstehenden beschrieben wurden. Insbesondere ist die Öffnung 18 an dem einteiligen, hülsenförmigen, magnetischen Rückschlusselement 34 durch das Verschlusselement 19 lösbar verschließbar. Des Weiteren kann vorzugsweise der Abschnitt 8B der gedruckten Leiterbahn 8A an der Außenseite des einteiligen, hülsenförmigen, magnetischen Rückschlusselements 34 vorgesehen, so dass der Abschnitt 8B mit einem außerhalb der Außenhülse 9 angeordneten elektrischen Kontakt 26 verbindbar ist.

Vorzugsweise weist der Generator 3 einen Außendurchmesser von kleiner etwa 1 cm auf, insbesondere von kleiner etwa 8 mm. Vorzugsweise weist der Generator 3 ohne die Fluidleitung 10 (d.h. vom Aufnahmeende 17 bis zum anschlussseitigen Ende 16) eine Länge von kleiner etwa 2 cm auf, insbesondere von kleiner etwa 1,7 cm. Vorzugsweise ist der Generator 3 ausgebildet, eine Leistung von etwa 0,05 Watt bis etwa 2 Watt, insbesondere von etwa 0,1 Watt, zu erzeugen. Insbesondere ist der Generator 3 ausgebildet, eine Beleuchtungsvorrichtung, insbesondere zumindest ein optisches Halbleiterelement (Leuchtdiode), mit elektrischer Energie zu versorgen.

Die Figur 3 zeigt ein erstes Ausführungsbeispiel einer medizinischen, insbesondere dentalen, Vorrichtung 1A mit dem fluidbetriebenen, dynamoelektrischen Wandler 3. Die medizinische, insbesondere dentale, Vorrichtung 1A umfasst einen Adapter oder eine Kupplungsvorrichtung 1A. Die Kupplungsvorrichtung 1A weist insbesondere ein lösbar mit einer Fluidquelle verbindbares Anschlussende 2A und ein davon beabstandetes Verbindungsende 2B zur lösbaren Verbindung mit einem medizinischen, insbesondere dentalen, Instrument, zum Beispiel einem Handgriffelement auf. Die Kupplungsvorrichtung 1A ist insbesondere als Drehkupplung ausgebildet, die einen zylindrischen oder hohlzylindrischen Kupplungszapfen 59 aufweist, der in eine entsprechende Aufnahme einer mit der Kupplungsvorrichtung 1A verbindbaren Vorrichtung einfügbar ist, so dass die Kupplungsvorrichtung 1A und die damit verbindbare Vorrichtung relativ zueinander drehbar sind. Der Adapter oder die Kupplungsvorrichtung 1A weisen vorzugsweise einen Körper 55 und eine den Körper zumindest teilweise umgebende Außenhülse 61 auf.

Der Generator 3 ist in einer Aufnahme 27 in der Kupplungsvorrichtung 1A angeordnet. Die Aufnahme 27 ist vorzugsweise an einem Ende der Kupplungsvorrichtung 1A vorgesehen, insbesondere an dem Anschlussende 2A. Die Aufnahme 27 ist vorzugsweise von dem Ende der Kupplungsvorrichtung 1A, an dem die Aufnahme 27 vorgesehen ist, insbesondere von dem Anschlussende 2A, zugänglich, besonders bevorzugt über eine Öffnung 51 an dem Ende oder Anschlussende 2A. Der Generator 3, insbesondere in Form einer austauschbaren Generatorpatrone, ist vorzugsweise durch die Öffnung 51 in die Aufnahme 27 einfügbar oder austauschbar. Vorzugsweise ist in der Aufnahme 27 zumindest ein Anschlag 52 vorgesehen, zum Beispiel ein Rücksprung oder eine Ringschulter, der einen Gegenanschlag 53 am Generator 3 kontaktiert.

Besonders bevorzugt ist der dynamoelektrischen Wandler 3 derart in der Aufnahme 27 angeordnet, dass die einteilig mit der Hülse oder Außenhülse 9 ausgebildete Fluidleitung 10 zumindest mit ihrem von dem dynamoelektrischen Wandler 3 abgewandten Ende über das Anschlussende 2A der medizinischen, insbesondere dentalen, Vorrichtung 1A, insbesondere der Kupplungsvorrichtung 1A, ragt. Die Fluidleitung 10 ist somit insbesondere direkt mit einer Fluidleitung lösbar verbindbar, vorzugsweise mit einer Fluidleitung, die mit einer Fluidquelle verbunden ist, zum Beispiel mit einer Fluidleitung eines Versorgungsschlauchs. Die einteilig mit der Hülse oder Außenhülse 9 ausgebildete Fluidleitung 10 bildet somit vorzugsweise zumindest einen Teil einer Kupplungseinheit 57 an dem Anschlussende 2A zur Verbindung mit einer Fluidquelle und / oder ein Kupplungselement zur Verbindung mit einer Fluidquelle. An der Kupplungseinheit 57 ist wahlweise noch zumindest eine weitere Leitungen 54 zur Übertragung eines Fluids vorgesehen, insbesondere in Form eines von dem Anschlussende 2A abstehenden Rohrs. Diese zumindest eine weitere Leitungen 54 ist entweder einteilig mit zumindest einem Teil des Körpers 55 der Kupplungsvorrichtung 1A ausgebildet oder sie ist in eine Aufnahme des Körpers 55 eingefügt. An dem Anschlussende 2A und / oder an der Kupplungseinheit 57 ist ein Dichtelement 56 vorgesehen, insbesondere in Form einer gummielastischen Dichtscheibe, welches insbesondere die Öffnung 51 und die Aufnahme 27 abdichtet.

Die Aufnahme 27 ist vorzugsweise zur Leitung des das Laufrad 5 antreibenden Fluids ausgebildet. Die Aufnahme 27 und die Hülse oder Außenhülse 9 des Generators 3 sind insbesondere derart angeordnet, dass zwischen ihnen ein Abstand oder Spalt gebildet ist, in dem das Fluid fließen kann, wahlweise das dem Laufrad 5 zufließende Fluid und / oder das vom Laufrad 5 abfließende Fluid. An die Aufnahme 27 anschließend und / oder mit der Aufnahme 27 verbunden ist eine Fluidleitung 49, die zur Aufnahme des Fluids, welches das Laufrad 5 passiert hat, ausgebildet ist. Die Fluidleitung 49 ist vorzugsweise mit einer fluidbetriebenen Antriebsvorrichtung für ein Behandlungswerkzeug und / oder mit einer Öffnung zur Abgabe des Fluids an die Umgebung verbunden oder verbindbar. Dazu ist die Fluidleitung 49 vorzugsweise mit einer Öffnung an dem Verbindungsende 2B, insbesondere an dem Kupplungszapfen 59, verbunden, über die das Fluid an die fluidbetriebene Antriebsvorrichtung und / oder an die Abgabeöffnung übertragbar ist.

In entsprechender Weise erstreckt sich zumindest eine weitere Leitung oder Bohrung 60 von der zumindest einen Leitung 54 am Anschlussende 2A durch den Adapter oder die Kupplungsvorrichtung 1A, insbesondere bis zu dem Verbindungsende 2B und / oder dem Kupplungszapfen 59, so dass darin ein Medium leitbar ist und insbesondere auf ein mit dem Verbindungsende 2B verbindbares Instrument übertragbar ist. Dazu ist die zumindest eine weitere Leitung 60 vorzugsweise mit einer Öffnung an dem Verbindungsende 2B, insbesondere an dem Kupplungszapfen 59, verbunden, über die das Medium abgebbar ist.

Ein in der medizinischen, insbesondere dentalen, Vorrichtung 1A vorgesehener elektrischer Kontakt 26 verbindet den Generator 3 mit einem elektrischen Verbraucher. Der elektrische Verbraucher ist wahlweise in dem Adapter oder in der Kupplungsvorrichtung 1A oder in einer, insbesondere über die Verbindungsvorrichtung 2B, damit verbindbaren Vorrichtung angeordnet. Der elektrische Kontakt 26 weist bevorzugt eine gedruckte Leiterbahn auf. Insbesondere weist der elektrische Kontakt 26 ein flexibles oder biegsames Trägerelement auf, insbesondere eine flexible oder biegsame Platine. Der elektrische Kontakt 26 ist vorzugsweise mit weiteren elektrischen Kontakten 58 an dem Verbindungsende 2B, insbesondere an dem Kupplungszapfen 59, verbunden, so dass die vom Generator 3 erzeugte elektrische Energie auf ein mit dem Verbindungsende 2B verbindbares Instrument übertragbar ist. Die elektrischen Kontakte 58 sind zum Beispiel als elektrische Kontaktring und / oder als Teil eines Schleifkontakts ausgebildet.

Gemäß einem bevorzugten Ausführungsbeispiel weisen der Adapter oder die Kupplungsvorrichtung 1A einen Körper 55 und eine an dem Körper befestigbare Außenhülse 61 auf, wobei der Körper 55 umfasst: Ein instrumentenseitiges Verbindungsende 2B und ein versorgungsseitiges Anschlussende 2A, wobei an dem Verbindungsende 2B ein Kupplungszapfen 59 zur lösbaren Verbindung mit einem medizinischen, insbesondere dentalen, Instrument und am Anschlussende 2A eine Kupplungseinheit 57 zur lösbaren Verbindung mit einer Versorgungs-, Steuer- und / oder Regeleinheit vorgesehen ist, und zumindest eine Leitung 26, 49, 60 zur Übertragung eines Mediums, von Energie und / oder Daten, die sich durch die Kupplungsvorrichtung 1A erstreckt oder die das Verbindungsende 2B und das Anschlussende 2A verbindet, so dass ein Medium, Energie und / oder Daten durch die Kupplungsvorrichtung 1A übertragbar ist / sind, wobei der Körper 55 der Kupplungsvorrichtung 1A zwei lösbar miteinander verbindbare Abschnitte 55A, 55B aufweist, und wobei an dem ersten Abschnitt 55A das Anschlussende 2A und an dem zweiten Abschnitt 55B das Verbindungsende 2B vorgesehen ist.

Bevorzugt ist eine Schnittfläche oder Schnittebene zwischen den beiden Abschnitten 55A, 55B der Kupplungsvorrichtung 1A gewinkelt, insbesondere im Wesentlichen rechtwinkelig, zur Längsachse der Kupplungsvorrichtung 1A angeordnet. Der Winkel zwischen der Schnittebene und der Längsachse der Kupplungsvorrichtung 1A ist insbesondere zumindest größer 0°. Vorzugsweise ist der zweite Abschnitt 55B im Wesentlichen durch den Kupplungszapfen 59 gebildet.

Bevorzugt sind der erste und der zweite Abschnitt 55A, 55B mittels einer Steckverbindung 62 miteinander verbindbar, welche an einem des ersten oder zweiten Abschnitts 55A, 55B ein Steckelement 62A und an dem anderen des ersten oder zweiten Abschnitts 55A, 55B eine Aufnahme 62B für das Steckelement 62A aufweist. Vorzugsweise weist zumindest eine Leitung 26, 49, 60 zur Übertragung eines Mediums, von Energie und / oder Daten einen ersten Leitungsabschnitt, der in dem ersten Abschnitt 55A vorgesehen ist, und einen zweiten Leitungsabschnitt, der in dem zweiten Abschnitt 55B vorgesehen ist, auf, wobei insbesondere der erste Leitungsabschnitt und der zweite Leitungsabschnitt durch die Steckverbindung 62 miteinander verbindbar sind. Vorzugsweise durchsetzt zumindest eine Leitung 49 zur Übertragung eines Mediums, von Energie und / oder Daten das Steckelement 62A und die Aufnahme 62B für das Steckelement 62A. Vorzugsweise ist an der Schnittfläche oder Schnittebene zwischen den beiden Abschnitten 55A, 55B und / oder an der Steckverbindung 62 und / oder an dem Verbindungsbereich zwischen dem ersten Leitungsabschnitt und dem zweiten Leitungsabschnitt zumindest ein Dichtelement 63 vorgesehen.

Vorzugsweise ist zumindest einer der beiden lösbar miteinander verbindbaren Abschnitte 55A, 55B des Adapters oder der Kupplungsvorrichtung 1A aus Kunststoff hergestellt. Vorzugsweise ist die Außenhülse 61 des Adapters oder der Kupplungsvorrichtung 1A aus Metall hergestellt.

Die Figur 4 ein zweites Ausführungsbeispiel einer medizinischen, insbesondere dentalen, Vorrichtung 1B in Form eines Handgriffelements, Handstücks oder Winkelstücks mit einem fluidbetriebenen, dynamoelektrischen Wandler. Die Vorrichtung 1B umfasst einen Kopfabschnitt 64, in dem insbesondere eine in Bewegung versetzbare Werkzeughalterung für ein Behandlungswerkzeug angeordnet ist.

Der in der Vorrichtung 1B vorgesehene fluidbetriebene, dynamoelektrische Wandler ist vorzugsweise ident mit dem im Vorstehenden beschriebenen fluidbetriebenen, dynamoelektrischen Wandler 3. Insbesondere ist der dynamoelektrischen Wandler 3 derart in einer Aufnahme 48 angeordnet, dass die einteilig mit der Hülse oder Außenhülse 9 ausgebildete Fluidleitung 10 zumindest mit ihrem von dem dynamoelektrischen Wandler 3 abgewandten Ende über das Anschlussende 2A der medizinischen, insbesondere dentalen, Vorrichtung 1B ragt. Die Fluidleitung 10 ist somit insbesondere direkt mit einer Fluidleitung lösbar verbindbar, vorzugsweise mit einer Fluidleitung, die mit einer Fluidquelle verbunden ist, zum Beispiel mit einer Fluidleitung eines Versorgungsschlauchs. Die einteilig mit der Hülse oder Außenhülse 9 ausgebildete Fluidleitung 10 bildet somit vorzugsweise zumindest einen Teil einer Kupplungseinheit 65 an dem Anschlussende 2A zur Verbindung mit einer Fluidquelle und / oder ein Kupplungselement zur Verbindung mit einer Fluidquelle. An der Kupplungseinheit 65 ist wahlweise noch zumindest eine weitere Leitungen 66 zur Übertragung eines Fluids vorgesehen, insbesondere in Form eines von dem Anschlussende 2A abstehenden Rohrs.

Alternativ weist der in der Vorrichtung 1B vorgesehene fluidbetriebene, dynamoelektrische Wandler 3' keine Fluidleitung 10 auf (siehe Figur 4). In diesem Fall ist eine in der Vorrichtung 1B vorgesehene und / oder daran befestigte Fluidleitung 67 ausgebildet, den Generator 3', insbesondere dessen Laufrad 5, mit Fluid zu versorgen. Die Fluidleitung 67 ist vorzugsweise an dem Anschlussende 2A vorgesehen und ragt insbesondere über das Anschlussende 2A. Zumindest ein Abschnitt der Fluidleitung 67 ist somit vorzugsweise an der Kupplungseinheit 65 vorgesehen oder bildet einen Teil der Kupplungseinheit 65. Die Kupplungseinheit 65 ist gemäß Figur 4 als Steckkupplung ausgebildet, sie kann jedoch alternativ und insbesondere unter Vorsehen des Generators 3' ohne Fluidleitung 10 als Drehkupplung mit einer Aufnahme für einen Kupplungszapfen eines Kupplungsgegenstücks ausgebildet sein.

Die Aufnahme 48 ist vorzugsweise zur Leitung des das Laufrad 5 antreibenden Fluids ausgebildet. Die Aufnahme 48 und die Hülse oder Außenhülse 9 des Generators 3' sind insbesondere derart angeordnet, dass zwischen ihnen ein Abstand oder Spalt gebildet ist, in dem das Fluid fließen kann, wahlweise das dem Laufrad 5 zufließende Fluid und / oder das vom Laufrad 5 abfließende Fluid. An die Aufnahme 48 anschließend und / oder mit der Aufnahme 48 verbunden ist eine Fluidleitung 49, die zur Aufnahme des Fluids, welches das Laufrad 5 passiert hat, ausgebildet ist. Die Fluidleitung 49 ist vorzugsweise mit einer fluidbetriebenen Antriebsvorrichtung, insbesondere einem Laufrad, für das Behandlungswerkzeug und / oder mit einer Öffnung zur Abgabe des Fluids auf die Behandlungsstelle verbunden.

Die medizinische, insbesondere dentale, Vorrichtung 1B weist vorzugsweise eine Beleuchtungsvorrichtung, insbesondere zumindest ein optisches Halbleiterelement oder zumindest eine Leuchtdiode auf, die mit dem Generator 3' elektrisch verbunden und / oder mit von dem Generator 3' erzeugter elektrischer Energie versorgbar ist. Die Beleuchtungsvorrichtung ist insbesondere in oder an oder angrenzend an den Kopfabschnitt 64 vorgesehen. Die Beleuchtungsvorrichtung ist besonders bevorzugt ringförmig um eine Werkzeugaufnahmeöffnung des Kopfabschnitts angeordnet. Die Beleuchtungsvorrichtung ist besonders bevorzug mit zumindest einer Öffnung zur Abgabe eines Fluids auf die Behandlungsstelle versehen.

## Patentansprüche

1. Medizinische, insbesondere dentale, Vorrichtung (1A, 1B) mit einem lösbar mit einer Fluidquelle verbindbaren Anschlussende (2A), mit einem fluidbetriebenen, dynamoelektrischen Wandler (3) und mit einer Aufnahme (27, 48) für den fluidbetriebenen, dynamoelektrischen Wandler (3), wobei der fluidbetriebene, dynamoelektrische Wandler (3) umfasst: Einen Rotor (4) mit einem durch ein Fluid in Drehung versetzbaren Laufrad (5) und zumindest einem Magnetelement (6), das mit dem Laufrad (5) verbunden ist, so dass das Magnetelement (6) durch das in Drehung versetzbare Laufrad (5) ebenfalls in Drehung versetzbar ist, einen Stator (7) mit einer elektrischen Wicklung (8), die mit dem in Drehung versetzbaren Magnetelement (6) derart zusammenwirkt, dass in der elektrischen Wicklung (8) elektrische Energie erzeugbar ist, eine Hülse (9), die zumindest Teile des Rotors (4) und / oder des Stators (7) umgibt, und eine einteilig mit der Hülse (9) des dynamoelektrischen Wandlers (3) ausgebildete Fluidleitung (10), welche zumindest mit ihrem von dem dynamoelektrischen Wandler (3) abgewandten Ende über das Anschlussende (2A) der medizinischen, insbesondere dentalen, Vorrichtung (1A, 1B) ragt, wobei der dynamoelektrische Wandler (3) mit der einteilig mit der Hülse (9) ausgebildeten Fluidleitung (10) durch Schieben oder Stecken in die Aufnahme (27, 48) der medizinischen, insbesondere dentalen, Vorrichtung (1A, 1B) einfügbar ist, **gekennzeichnet durch**
zumindest eine an der Außenseite der Hülse (9) des dynamoelektrischen Wandlers (3) vorgesehene, vorzugsweise ring- oder kreisbogenförmige, Nut (13), die mit der einteilig mit der Hülse (9) des dynamoelektrischen Wandlers (3) ausgebildeten Fluidleitung (10) verbunden ist, so dass ein in der Fluidleitung (10) leitbares Fluid in der Nut (13) weiterleitbar ist.

2. Medizinische, insbesondere dentale, Vorrichtung (1A, 1B) nach Anspruch 1, **dadurch gekennzeichnet, dass**
die Hülse (9) des dynamoelektrischen Wandlers (3) zumindest ein Bohrung (11) aufweist, die mit der Fluidleitung (10) und der zumindest einen Nut (13) verbunden ist und die ausgebildet ist, ein in der Fluidleitung (10) leitbares Fluid zu dem Laufrad (5) des dynamoelektrischen Wandlers (3) zu leiten.

3. Medizinische, insbesondere dentale, Vorrichtung (1A, 1B) nach Anspruch 2, **gekennzeichnet, durch**
eine mit der Bohrung (11) und / oder mit der einteilig mit der Hülse (9) ausgebildeten Fluidleitung (10) verbundene erste Auslassöffnung (12), so dass das Fluid in die an der Außenseite der Hülse (9) vorgesehene Nut (13) leitbar ist.

4. Medizinische, insbesondere dentale, Vorrichtung (1A, 1B) nach einem der vorstehenden Ansprüche, **gekennzeichnet durch**
ein einteiliges, hülsenförmiges, magnetisches Rückschlusselement (34) zum Konzentrieren und Lenken der magnetischen Feldlinien des zumindest einen Magnetelements (6) durch die elektrische Wicklung (8), wobei das Rückschlusselement (34) als Teil der Hülse (9) ausgebildet ist.

5. Medizinische, insbesondere dentale, Vorrichtung (1A, 1B) nach einem der vorstehenden Ansprüche, **gekennzeichnet durch**
zumindest eine an der Außenseite der Hülse (9) des dynamoelektrischen Wandlers (3) vorgesehene Einlassöffnung (14), so dass das Fluid von der Außenseite der Hülse (9), insbesondere von der Nut (13), in das Innere der Hülse (9), insbesondere zu dem Laufrad (5), leitbar ist.

6. Medizinische, insbesondere dentale, Vorrichtung (1A, 1B) nach einem der vorstehenden Ansprüche, **gekennzeichnet durch**
zumindest eine an der Außenseite der Hülse (9) des dynamoelektrischen Wandlers (3) vorgesehene zweite Auslassöffnung (15), welche ausgebildet ist, das Fluid nach Passage des Laufrads (5) an die Außenseite der Hülse (9) zu leiten.

7. Medizinische, insbesondere dentale, Vorrichtung (1A, 1B) nach einem der vorstehenden Ansprüche, **dadurch gekennzeichnet, dass**
die Hülse (9) länglich ausgebildet ist und ein anschlussseitiges Ende (16), an dem einteilig die Fluidleitung (10) vorgesehen ist, und ein Aufnahmeende (17) aufweist, an dem eine Öffnung (18) vorgesehen ist, durch die der Rotor (4) und die elektrische Wicklung (8) des Stators (7) in die Hülse (9) einfügbar sind.

8. Medizinische, insbesondere dentale, Vorrichtung (1A, 1B) nach Anspruch 7, **dadurch gekennzeichnet, dass**
die Öffnung (18) am Aufnahmeende (17) der Hülse (9) durch ein Verschlusselement (19) lösbar verschlossen ist, an dem eine Lagerstelle (20) zur drehbaren Lagerung des Magnetelements (6) des Rotors (4) und / oder ein Trägerelement (21) für die elektrische Wicklung (8) vorgesehen ist / sind.

9. Medizinische, insbesondere dentale, Vorrichtung (1A, 1B) nach einem der vorstehenden Ansprüche, **dadurch gekennzeichnet, dass**
der Außendurchmesser der einteilig mit der Hülse (9) ausgebildete Fluidleitung (10) geringer ist als der Außendurchmesser zumindest eines Abschnitts der Hülse (9).

10. Medizinische, insbesondere dentale, Vorrichtung (1A, 1B) nach einem der vorstehenden Ansprüche, **dadurch gekennzeichnet, dass**
im Inneren einer das zumindest eine Magnetelement (6) des Rotors (4) aufnehmenden Rotorhülse (22) und / oder im Inneren des Laufrads (5) ein Lagerstelle (23) und / oder ein Lager (24) zur drehbaren Lagerung des Rotors (4) und / oder des Laufrads (5) vorgesehen ist / sind.

11. Medizinische, insbesondere dentale, Vorrichtung (1A, 1B) nach einem der vorstehenden Ansprüche, **gekennzeichnet durch**
eine in einer Wand, insbesondere in einer anschlussseitigen Endwand (16A), der Hülse (9) des dynamoelektrischen Wandlers (3) vorgesehene Lagerstelle (25) zur drehbaren Lagerung des Laufrads (5) und / oder des Rotors (4).

12. Medizinische, insbesondere dentale, Vorrichtung (1A, 1B) nach einem der vorstehenden Ansprüche, **dadurch gekennzeichnet, dass**
die elektrische Wicklung (8) des Stators (7) eine gedruckte Leiterbahn (8A) aufweist.

13. Medizinische, insbesondere dentale, Vorrichtung (1A, 1B) nach Anspruch 12, **dadurch gekennzeichnet, dass**
die gedruckte Leiterbahn (8A) einen Abschnitt (8B) aufweist, der an der Außenseite der Hülse (9) des dynamoelektrischen Wandlers (3) oder an der Außenseite des Verschlusselements (19) der Hülse (9) vorgesehen ist, so dass der Abschnitt (8B) mit einem außerhalb der Hülse (9) angeordneten elektrischen Kontakt (26) verbunden ist.

14. Medizinische, insbesondere dentale, Vorrichtung (1A, 1B) nach einem der vorstehenden Ansprüche, **dadurch gekennzeichnet, dass**
an dem Anschlussende (2A) der medizinischen, insbesondere dentalen, Vorrichtung (1A, 1B) die Aufnahme (27) für den dynamoelektrischen Wandler (3) vorgesehen ist, die derart bemessen, dass, wenn der dynamoelektrische Wandler (3) in der Aufnahme (27) aufgenommen ist und seine Betriebsposition einnimmt, die einteilig mit der Hülse (9) des dynamoelektrischen Wandlers (3) ausgebildete Fluidleitung (10) zumindest mit ihrem von dem dynamoelektrischen Wandler (3) abgewandten Ende über das Anschlussende (2A) ragt.

15. Medizinische, insbesondere dentale, Vorrichtung (1A, 1B) nach einem der vorstehenden Ansprüche, **dadurch gekennzeichnet, dass**
die medizinische, insbesondere dentale, Vorrichtung (1A, 1B) einen Adapter oder eine Kupplungsvorrichtung (1A) aufweist, der / die das lösbar mit einer Fluidquelle verbindbare Anschlussende (2A) und ein davon beabstandetes Verbindungsende (2B) zur lösbaren Verbindung mit einem medizinischen, insbesondere dentalen, Handgriffelement umfassen.

## Claims

1. A medical, in particular dental, device (1A, 1B) with a connecting end (2A) that can be releasably connected to a fluid source, with a fluid-operated dynamoelectric converter (3), and with a receptacle (27, 48) for the dynamoelectric converter (3), wherein the fluid-operated dynamoelectric converter (3) comprises: a rotor (4) having an impeller (5) that can be put into rotation by a fluid and at least one magnetic element (6) which is connected to the impeller (5), so that the magnetic element (6) can also be put into rotation by the rotatable impeller (5), a stator (7) with an electric winding (8) which cooperates with the rotatable magnetic element (6), so that electricity can be generated in the electric winding (8), a sleeve (9), which surrounds at least parts of the rotor (4) and/or of the stator (7), and a fluid line (10) which is designed in one piece with the sleeve (9) of the dynamoelectric converter (3) and which protrudes at least at its end which faces away from the dynamoelectric converter (3) beyond the connecting end (2A) of the medical, in particular dental, device (1A, 1B), wherein the dynamoelectric converter (3) with the fluid line (10) which is designed in one piece with the sleeve (9) can be inserted into the receptacle (27, 48) of the medical, in particular dental, device (1A, 1B) by displacement or plugging it in, **characterized by** at least one groove (13), preferably ring-shaped or arc-shaped, which is provided on the outside of the sleeve (9) of the dynamoelectric converter (3) and which is connected to the fluid line (10) which is designed in one piece with the sleeve (9) of the dynamoelectric converter (3), so that a fluid that can be conducted in the fluid line (10) can be conducted further in the groove (13).

2. The medical, in particular dental, device (1A, 1B) according to claim 1, **characterized in that**
the sleeve (9) of the dynamoelectric converter (3) comprises at least one bore (11) which is connected to the fluid line (10) and to the at least one groove (13) and is designed to conduct a fluid that can be conducted in the fluid line (10) to the impeller (5) of the dynamoelectric converter (3).

3. The medical, in particular dental, device (1A, 1B) according to claim 2, **characterized by**
a first outlet opening (12) that is connected to the bore (11) and/or to the fluid line (10) designed in one piece with the sleeve (9), so that the fluid can be conducted to the groove (13) which is provided on the outside of the sleeve (9).

4. The medical, in particular dental, device (1A, 1B) according to any one of the preceding claims, **characterized by**
a one-piece, sleeve-shaped magnetic yoke element (34) for concentrating and deflecting the magnetic field lines of the at least one magnetic element (6) through the electric winding (8), wherein the yoke element (34) is designed as a part of the sleeve (9).

5. The medical, in particular dental, device (1A, 1B) according to any one of the preceding claims, **characterized by**
at least one inlet opening (14) provided on the outside of the sleeve (9) of the dynamoelectric converter (3), so that the fluid can be conducted from the outside of the sleeve (9), in particular from the groove (13) into the interior of the sleeve (9), in particular to the impeller (5).

6. The medical, in particular dental, device (1A, 1B) according to any one of the preceding claims, **characterized by**
at least one second outlet opening (15) which is provided on the outside of the sleeve (9) of the dynamoelectric converter (3) and is designed to conduct the fluid to the outside of the sleeve (9) after passage of the impeller (5).

7. The medical, in particular dental, device (1A, 1B) according to any one of the preceding claims, **characterized in that**
the sleeve (9) is designed to be elongated and comprises an connecting end (16) on which the fluid line (10) is provided in one piece and a receiving end (17) on which an opening (18) is provided through which the rotor (4) and the electric winding (8) of the stator (7) can be inserted into the sleeve (9).

8. The medical, in particular dental, device (1A, 1B) according to claim 7, **characterized in that**
the opening (18) on the receiving end (17) of the sleeve (9) is releasably closed by a closure element (19) on which a bearing site (20) for rotatable bearing support of the magnetic element (6) of the rotor (4) and/or a carrier element (21) for the electric winding (8) is/are provided.

9. The medical, in particular dental, device (1A, 1B) according to any one of the preceding claims, **characterized in that**
the outside diameter of the fluid line (10) which is designed in one piece with the sleeve (9) is smaller than the outside diameter of at least a section of the sleeve (9).

10. The medical, in particular dental, device (1A, 1B) according to any one of the preceding claims, **characterized in that**
in the interior of a rotor sleeve (22) which accommodates the at least one magnetic element (6) of the rotor (4) and/or in the interior of the impeller (5) a bearing surface (23) and/or a bearing (24) is/are provided for a rotatable bearing support of the rotor (4) and/or of the impeller (5).

11. The medical, in particular dental, device (1A, 1B) according to any one of the preceding claims, **characterized by**
a bearing surface (25) which is provided in a wall, in particular in an connecting end wall (16A) of the sleeve (9) of the dynamoelectric converter (3) for rotatable bearing support of the impeller (5) and/or of the rotor (4).

12. The medical, in particular dental, device (1A, 1B) according to any one of the preceding claims, **characterized in that**
the electric winding (8) of the stator (7) comprises a printed conductor (8A).

13. The medical, in particular dental, device (1A, 1B) according to claim 12, **characterized in that**
the printed conductor (8A) comprises a section (8B), which is provided on the outside of the sleeve (9) of the dynamoelectric converter (3) or on the outside of the closure element (19) of the sleeve (9), so that the section (8B) is connected to an electric contact (26) arranged outside of the sleeve (9).

14. The medical, in particular dental, device (1A, 1B) according to any one of the preceding claims, **characterized in that**
a receptacle (27) for the dynamoelectric converter (3) is provided on the connecting end (2A) of the medical, in particular dental, device (1A, 1B), the receptacle (27) being of such dimensions that when the dynamoelectric converter (3) is accommodated in the receptacle (27) and is in its operating position, the fluid line (10), which is designed in one piece with the sleeve (9) of the dynamoelectric converter (3), protrudes beyond the connecting end (2A) at least at its end facing away from the dynamoelectric converter (3).

15. The medical, in particular dental, device (1A, 1B) according to any one of the preceding claims, **characterized in that**
the medical, in particular dental, device (1A, 1B) comprises an adapter or a coupling device (1A), which comprises the connecting end (2A) that is releasably connectable to a fluid source and a connecting end (2B) at a distance from the connecting end (2A) for releasable connection to a medical, in particular dental, handle element.

## Revendications

1. Dispositif médical, en particulier dentaire (1A, 1B) avec une extrémité de raccordement (2A) pouvant être raccordée de manière détachable à une source de fluide, avec un convertisseur dynamoélectrique (3) actionné par fluide et avec un logement (27, 48) pour le convertisseur dynamoélectrique (3) actionné par fluide, dans lequel le convertisseur dynamoélectrique (3) actionné par fluide comprend: un rotor (4) avec une roue mobile (5) pouvant être mise en rotation par un fluide et au moins un élément magnétique (6) relié à la roue mobile (5) de sorte que l'élément magnétique (6) peut être également mis en rotation par la roue mobile (5) pouvant être mise en rotation, un stator (7) avec un enroulement électrique (8) qui coopère avec l'élément magnétique (6) pouvant être mis en rotation de manière à pouvoir générer de l'énergie électrique dans l'enroulement électrique (8), un manchon (9) qui entoure au moins des parties du rotor (4) et/ou du stator (7), et une conduite de fluide (10) formée d'une seule pièce avec le manchon (9) du convertisseur dynamoélectrique (3), laquelle fait saillie au moins avec son extrémité tournant le dos au convertisseur dynamoélectrique (3) par rapport à l'extrémité de raccordement (2A) du dispositif médical, en particulier dentaire (1A, 1B), dans lequel le convertisseur dynamoélectrique (3) avec la conduite de fluide (10) formée d'une seule pièce avec le manchon (9) peut être inséré par glissement ou enfichage dans le logement (27, 48) du dispositif médical, en particulier dentaire (1A, 1B), **caractérisé par** au moins une rainure (13), de préférence en forme d'anneau ou d'arc de cercle, prévue sur le côté extérieur du manchon (9) du convertisseur dynamoélectrique (3), laquelle est en liaison avec la conduite de fluide (10) réalisée d'une seule pièce avec le manchon (9) du convertisseur dynamoélectrique (3) de sorte qu'un fluide pouvant être conduit dans la conduite de fluide (10) peut être conduit plus loin dans la rainure (13) .

2. Dispositif médical, en particulier dentaire (1A, 1B) selon la revendication 1, **caractérisé en ce que**
le manchon (9) du convertisseur dynamoélectrique (3) présente au moins une forure (11) qui est en liaison avec la conduite de fluide (10) et l'au moins une rainure (13) et qui est réalisée pour conduire un fluide pouvant être conduit dans la conduite de fluide (10) vers la roue mobile (5) du convertisseur dynamoélectrique (3).

3. Dispositif médical, en particulier dentaire (1A, 1B) selon la revendication 2, **caractérisé par**
une première ouverture de sortie (12) en liaison avec la forure (11) et/ou avec la conduite de fluide (10) formée d'une seule pièce avec le manchon (9), de sorte que le fluide peut être conduit dans la rainure (13) prévue sur le côté extérieur du manchon (9).

4. Dispositif médical, en particulier dentaire (1A, 1B) selon l'une des revendications précédentes, **caractérisé par**
un élément de blindage magnétique (34) d'une seule pièce en forme de manchon pour concentrer et guider les lignes de champ magnétique de l'au moins un élément magnétique (6) à travers l'enroulement magnétique (8), dans lequel l'élément de blindage (34) est réalisée en tant que partie du manchon (9).

5. Dispositif médical, en particulier dentaire (1A, 1B) selon l'une des revendications précédentes, **caractérisé par**
au moins une ouverture d'entrée (14) prévue sur le côté extérieur du manchon (9) du convertisseur dynamoélectrique (3) de sorte que le fluide peut être conduit depuis le côté extérieur du manchon (9), en particulier depuis la rainure (13), dans l'intérieur du manchon (9), en particulier vers la roue mobile (5).

6. Dispositif médical, en particulier dentaire (1A, 1B) selon l'une des revendications précédentes, **caractérisé par**
au moins une deuxième ouverture de sortie (15) prévue sur le côté extérieur du manchon (9) du convertisseur dynamoélectrique (3), laquelle est réalisée pour conduire le fluide, après le passage par la roue mobile (5), vers le côté extérieur du manchon (9).

7. Dispositif médical, en particulier dentaire (1A, 1B) selon l'une des revendications précédentes, **caractérisé en ce que**
le manchon (9) est réalisé de manière oblongue et présente une extrémité (16) côté raccordement à laquelle la conduite de fluide (10) est prévue d'une seule pièce et une extrémité de logement (17) à laquelle une ouverture (18) est prévue à travers laquelle le rotor (4) et l'enroulement électrique (8) du stator (7) sont insérables dans le manchon (9).

8. Dispositif médical, en particulier dentaire (1A, 1B) selon la revendication 7, **caractérisé en ce que**
l'ouverture (18) à l'extrémité de logement (17) du manchon (9) est fermée de manière détachable par un élément de fermeture (19) au niveau duquel un emplacement de logement (20) pour le logement rotatif de l'élément magnétique (6) du rotor (4) et/ou un élément de support (21) pour l'enroulement électrique (8) est/sont prévu(s).

9. Dispositif médical, en particulier dentaire (1A, 1B) selon l'une des revendications précédentes, **caractérisé en ce que**
le diamètre extérieur de la conduite de fluide (10) formée d'une seule pièce avec le manchon (9) est inférieur au diamètre extérieur d'au moins une partie du manchon (9).

10. Dispositif médical, en particulier dentaire (1A, 1B) selon l'une des revendications précédentes, **caractérisé en ce que**,
à l'intérieur d'un manchon de rotor (22) réceptionnant l'au moins un élément magnétique (6) du rotor (4) et/ou à l'intérieur de la roue mobile (5), un emplacement de logement (23) et/ou un palier (24) pour le logement rotatif du rotor (4) et/ou de la roue mobile (5) est/sont prévu(s).

11. Dispositif médical, en particulier dentaire (1A, 1B) selon l'une des revendications précédentes, **caractérisé par**
un emplacement de logement (25) prévu dans une paroi, en particulier dans une paroi d'extrémité (16A) côté raccordement, du manchon (9) du convertisseur dynamométrique (3) pour le logement rotatif de la roue mobile (5) et/ou du rotor (4).

12. Dispositif médical, en particulier dentaire (1A, 1B) selon l'une des revendications précédentes, **caractérisé en ce que**
l'enroulement électrique (8) du stator (7) présente une piste conductrice imprimée (8A).

13. Dispositif médical, en particulier dentaire (1A, 1B) selon la revendication 12, **caractérisé en ce que**
la piste conductrice imprimée (8A) présente une partie (8B) qui est prévue sur le côté extérieur du manchon (9) du convertisseur dynamoélectrique (3) ou sur le côté extérieur de l'élément de fermeture (19) du manchon (9) de sorte que la partie (8B) est reliée à un contact électrique (26) disposé à l'extérieur du manchon (26).

14. Dispositif médical, en particulier dentaire (1A, 1B) selon l'une des revendications précédentes, **caractérisé en ce que**,
au niveau de l'extrémité de raccordement (2A) du dispositif médical, en particulier dentaire (1A, 1B), le logement (27) pour le convertisseur dynamoélectrique (3) est prévu, lequel est dimensionné de telle sorte que, lorsque le convertisseur dynamoélectrique (3) est réceptionné dans le logement (27) et adopte sa position de fonctionnement, la conduite de fluide (10) formée d'une seule pièce avec le manchon (9) du convertisseur dynamoélectrique (3) fait saillie, au moins avec son extrémité tournant le dos au convertisseur dynamoélectrique (3), par rapport à l'extrémité de raccordement (2A).

15. Dispositif médical, en particulier dentaire (1A, 1B) selon l'une des revendications précédentes, **caractérisé en ce que**
le dispositif médical, en particulier dentaire (1A, 1B) présente un adaptateur ou un dispositif de couplage (1A) qui comprend l'extrémité de raccordement (2A) pouvant être raccordée de manière détachable à une source de fluide et une extrémité de liaison (2B) espacée par rapport à celle-ci pour le raccordement détachable à un élément de poignée médical, en particulier dentaire.
